# EUROPEAN PATENT APPLICATION

(11) **EP 2 098 589 A2**
(43) Date of publication of application: **09.09.2009**
(21) Application number: 09001915.9
(22) Date of filing: 11.02.2009
(51) Int. Cl.: C12N 5/08, A61P 35/00, A61K 39/00

(54) **Immune cell modification method and cell modification device**

(30) Priority: 15.02.2008 JP 2008034313; 11.07.2008 JP 2008181172
(71) Applicant: KIST-Europe Forschungsgesellschaft mbH, 66123 Saarbrücken (DE)
(72) Inventor: Steinfeld, Ute, Dr., 66386 St. Ingbert (DE); Lee, Hyeck-Hee, Dr., 66386 St. Ingbert (DE)
(74) Representative: Pfenning, Meinig & Partner GbR

(57) **Abstract**

The present invention relates to a cell modification method for modifying human or mammal immune cells, especially effector cells outside the human or mammal body, wherein in a first step at least one encapsulated substance, preferably an active pharmaceutical ingredient, is introduced in and/or arranged on isolated human or mammal immune cells and wherein in a second step prior to or after the introduction of said at least one substance the cytotoxic effector function of the isolated immune cells is enhanced. The present invention also relates to a correspondingly modified human or mammal immune cell and to a cell modification device.

## Description

The present invention relates to a cell modification method and a cell modification device which modify cellular components of human or animal blood, especially cells of the body's natural immune defense, such as for example T-cells, NK cells, monocytes, macrophages or microglia, such that the cells exert either a therapeutic effect, for example against cancerous diseases, e.g. of the lung or the pancreas or the ovaries, the breast or brain, or against other diseases, or a diagnostic effect, after their introduction into the human or animal body, especially the mammal body. The present invention also relates to accordingly modified immune cells.

The main aim of the present invention is to use immune cells as target-oriented transporters of active substances or agents (for example medicaments, RNAi, ...) and/or diagnostics. In order to improve the target-oriented, autonomous transport, i. e. the targeting of diseased tissue, retargeting strategies such as bi-specific antibodies, genetically transformed cells related to TCR or others are used. The immune cells are, due to their usage as living transporters, heading for the metastases (e. g. tumours) and exert firstly an intrinsic cytotoxic function. The agent or the active substance is then released for fortification. The releasing is triggered by specific internal or externally applied physical or physiological factors such as pH, temperature or release in a time-dependent manner such as by degradation of the particles/substances or of an outer shell caused for example by enzymes in a cell's cytoplasm or lysosomes/ endosomes or by pH. The agent or active substance increases the natural effect of the immune cell (intrinsic cytolytical/cytotoxic features).

Here and in the following sections the cellular components described are also designated in short as cells. The cells can also involve for example other differentiated, naturally occurring cells of the human or animal body or not yet differentiated cells, such as stem cells. The therapeutic effect of the modified cells, which are then introduced as a medicament in the mammal body, can involve for example a controlled active ingredient release or tissue regeneration or the like.

The therapeutic effect of the so modified cells can include:
- releasing of an agent or active substance such as a cytostatic drug and/or angiogenesis inhibitors,
- releasing of antibody based therapeutic agents,
- releasing of therapeutic DNA for transfection,
- releasing of RNAi-based therapeutic agents, which are selectively aimed to genes and viruses causing diseases; and/or
- releasing of chemokines such as cytokines or proteins peptides or glycoproteins, i.e. regulators that promote further cells of the immune system or cause apoptosis like TNF alfa for example; or, for diagnostic purposes:
- loading of the cells with contrast agents such as e. g. ferromagnetic particles (e. g. for NMR-diagnostics).
Therapeutic or diagnostic agents can also be used together, for example either combined in immune cells or by applying immune cell fractions which are either loaded with therapeutic agents or withdiagnostic agents.

Methods for medical treatment through active ingredients have been known for a long time. In these methods the active ingredient is usually delivered to the whole human or animal body. The active ingredient can be administered for example orally or by injection; it then distributes itself uniformly in the whole human or animal organism. The decisive disadvantage of the previous treatment methods is to be seen in that unaffected regions of the human or animal body can also be affected by the active ingredients and that only a small part of the active ingredient can act in the target regions. Thus, correspondingly high active ingredient doses are avoidable.

The task of the present invention is thus to make a method and a device available that permits human or animal cells to be modified such that these cells, when being reintroduced into the human or animal body, are delivered to actively targeted, desired body parts or cells and exert a therapeutic or diagnostic effect there. By modifying the animal or human cells in this way, it is possible for example to fight diseases with low doses in a targeted way or to build up and strengthen tissue in a targeted way without affecting uninvolved regions of the body and/or detected diseased sites within the body for example tumor and metastasis, o.a..

The objective of the present invention is solved by a method according to claim 1, by a modified immune cell according to claim 10 and by a device according to claim 13. Advantageous embodiments are described in the dependent claims. Uses according to the invention are described in claims 14 and 15.

The basic aspect of the solution of the above mentioned problem provided by the present invention is to provide a cell modification method wherein in a first step one encapsulated substance (or more than one encapsulated substance) preferably an active pharmaceutical ingredient, is introduced in and/or arranged on isolated human or mammal immune cells and wherein in a second step prior to or after the introduction of said at least one encapsulated substance, the cytotoxic effector function of the isolated immune cells is enhanced and/or the targeting capabilities or the target finding capabilities, respectively, of the isolated immune cells are enhanced.

The encapsulated substance can be introduced into the cell; however, it is also possible to arrange, to connect or to adhere this substance on/with/to the outer surface of the cell. The latter can be realized for example with help of electrostatic interaction techniques, with help of (e.g. bispecific, monospecific or trispecific) antibodies or molecular imprinting the surface of the encapsulated substance (in order to allow the substance to be arranged on or fixed to the cell's surface).

The meaning of "enhancing the cytotoxic effector function of the isolated immune cells and/or enhancing the targeting capabilities of the isolated immune cells" is to be understood in a very general way within the present invention: Each approach of the cellular immunotherapy, especially the adoptive immunotherapy, which enhances the capabilities of the immune cells to be modified according to the present invention to recognize and/or to find target cells in the human or animal body (e.g. the tumor cells) is to be understood as lying within the scope of the corresponding expression. In other words, in order to perform the second step (or in order to effect the corresponding modification within the immune cells modified according to the present invention) each retargeting strategy used to enhance the recognition of the diseased target cells in the human or animal body can be used. Especially, the meaning of "enhancing the cytotoxic effector function" is to be understood also in a way that it includes a modification of the immune cell such that the cytotoxic effector function of these immune cells (carrier cells) at the target (e.g. a tumor) will be enhanced when the carrier cell reaches said diseased target. Especially it is possible, that the cytotoxic effector function can be enhanced by the releasing of the encapsulated substance at the target. As is further described below, the enhancement of the targeting capabilities of the isolated immune cells within the second step can be realized in a number of different ways: E.g. a retargeting strategy using T-cells with chimeric receptors can be used or a combination of the immune cells with bi-specific antibodies or the like can be realized. Therein, it can be advantageous to modify the immune cells such that the cytotoxic effector function of the so modified/treated cells is enhanced at a diseased side following reinfusion into the body.

The immune cells to be modified, which can be used in the present invention can be any type of immune cells used in adoptive immunotherapy, such as e.g. autologous or allogenic cells. For example, T-cells can be modified; beside patient's own or donor cells, also cell lines can be used, such as cytotoxic T-cell lines like TALL-104 cells or C CURE 709 cells. Also NK cells can be modified, such as NK cell lines like NK-92.

The cells to be modified can therefore be T-cells (monoclonal as well as polyclonal T-cells), especially retargeted polyclonal T-cells (retargeted as described above or by other means), tumor antigen specific T-lymphocytes, genetically modified T-lymphocytes or cytotoxic T-cells.

This second step can be performed prior to said first step or also alternatively after the first step. The enhancement of the cytotoxic effector function of the immune cells which previously had been isolated from the human or animal body can be realized in a number of different ways which is subsequently described in more detail.

Basically, the cell modification method according to the present invention can be realized with the help of well-known cell isolation kits such as the Rosettesep^{™} kit of Stem Cell Technologies, 5070 West Seventh Avenue, Vancouver, BC, Canada in order to isolate specific immune cells. The isolated immune cells are loaded with therapeutic agents or active substances or with diagnostic substances. This can be done by co-incubation; however, the loading can also be supported by electroporation. Alternatively, however, the present cell modification method can also be performed with a cell modification device according to the present invention, which is also outlined further below.

Focus of the method to enhance the effector function of immune cells for their use in the areas of applied immunology or oncology and/or for the diagnosis of tumors according to the invention is the enhancement of or the increase in the disease fighting effect of immune cells, such as cytotoxic T-cells, especially (preferably activated) cytotoxic T-lymphocytes, such as natural killer cells (NK cells), such as monocytes or such as monocyte-derived macrophages, such as microglia cells. Accordingly modified immune cells can be used in adoptive immunotherapy or in diagnosis.

Such effector cells are, as part of the human or animal immune system, capable of recognizing diseased tissue or tumor tissue and to lyse such tissue with the help of their cytotoxic effector function. The natural capability of such cells to migrate during a viral or anti-tumoral answer, actively in non-lymphatic tissue and to infiltrate diseased tissue predestines these cells to be used for the autonomous and active substance targeting or for the diagnostics. Tumor escape mechanisms, such as angery, apoptosis induction of the immune cells or suppression of the effector function, often prevent the immune system from destroying the tumor itself.

In order to enhance this effector function and to prevent the tumor escape mechanisms, the above described two-step approach is used in which in addition one or more temporally encapsulated substances, especially active pharmaceutical ingredients, are introduced in the immune cells or arranged on said immune cells. The encapsulation of the substances is realized thus that the substance will not be released immediately in or on the immune cell, but after a period of at least some hours, preferably a period of between one day and 25 days. This is normally necessary for the immune cells to reach the target tissue without being harmed or even destroyed by the loaded substance before the immune cell can reached the targeted tissue and to profit of the intrinsic cytotoxic action to destroy tumor cells.

The size of the encapsulated substance or the encapsulated medicament is between approximately 10 nm and 1 µm. For example, such an encapsulation can be realized with help of (e.g. by introducing the substance in) liposomes, encapsulated liposomes, e.g. with alginate (e.g. Ba-alginate), multi-laminar liposomes, vesosomes, virosomes or in microspheres comprising or consisting of porous materials, such as polylactide or Polymer Polyglycolic-Lactic Acid (PGLA), amylum, apatite and/or pressed polymers (with or without an external coating or the like)Dextran, Agraose, Albumin, Chitosan, Silica or Hollow silica particles, Polyethylenimin, Polyalkylcyanoacrylat, Polymethylmethacrylateparticles, core shell nanoprticles, dendrimers and dendronised polymers of which the core molecule is usually an amine or a sugar, with several identical bonding sites for shell molecules available on the surface. The shells usually alternate between an acid and an amine.

The above-mentioned bodies (liposomes, .........) used for the encapsulation of the medicament/substance can also be modified bodies: The bodies (liposomes, .........) can be modified with help of antibodies or fragments of antibodies or peptides, respectively (in addition to the loading of a substance or the introduction of the substance, respectively), in order to secure a recognition of a diseased cell (especially a tumor cell), a binding to such a cell and/or the incorporation into such a cell. In this case, the immune cells to be modified are loaded with the correspondingly modified bodies/liposomes, when the substance/medicament is released, the membrane integrity of the carrier cell is suspended, the liposomes leave the cells and can then bind to the cancer cells or can then be incorporated into these cancer cells, respectively (e.g. with help of receptors or the like).

In order to enhance the incorporation efficiency into the immune cell (carrier cell) the encapsulation can be modified with ligands to receptors expressed on the immune cell's surface such as so-called cell penetrating peptides (CPPs). CPPs are short polycationic sequences simplifying the incorporation of peptides or proteins which carry such sequences. If these CPPs are bound covalently to a nano particle or a liposome, the enhance the incorporation into the cell. Examples for such CPPs are TAT and penetrine, peptides of the HIV virus.

Releasing of encapsulated active substances or agents, modified in order to improve the incorporation in cancer cells, is especially qualified to incorporate therapeutic DNA for transfection or RNAi-based therapeutic agents in the carrier cell.

The efficiency of this approach can be further enhanced by using bi-specific liposomes for the loading into the carrier cell: Especially, the latter liposomes can be, on the one hand, loaded with the medicament and/or therapeutic DNA and/or RNAi based therapeutics or the like and, on the other hand, be modified with antibodies, with derivates thereof (against tumor-specific surface markers especially rapidly internalizing tumor-associated antigens (TAA)) and/or by peptides (for the incorporation based on receptors or the like). It is also possible to use liposomes loaded with the substance/medicament, which release the substance/medicament earlier and which have the only purpose to kill the carrier cell in order to allow the liposomes described above to be released from the cell in a more easy way. The releasing of the substance/medicament from the liposomes described above can be realized temporarily delayed compared to the releasing of the liposomes in order to allow the medicament to be released exactly at the diseased tissue (e.g. tumor).

In the cell modification method for the manufacture of a medicament or a diagnostics, immune cells (such as precursor cells or also terminally differentiated effector cells) can for example be drawn from or isolated of peripheral blood of the mammal or tumor tissue of the mammal. After preparing and isolating these immune cells such that they are ready to be loaded with the encapsulated substance, the second step, performed prior to or after the loading of the substance, in order to enhance the effector function and/or the targeting capabilities of the (isolated) immune cells (preferably following reinfusion at the target site) can be performed in a number of different ways to enhance the capability of the modified cells to find the diseased tissue or the tumor tissue or the like:

A first possibility to realize said second step of the enhancement of the cytotoxic effector function and/or of the targeting capabilities is to introduce immune cell receptor genes (such as T-cell receptor genes, TCR genes) from a tumor-associated antigen (TAA)-specific immune cell (especially a TAA-specific T-cell) in the isolated immune cells. Preferably such introduction can be performed by genetic transfer. How a corresponding genetic transfer can be performed, is well known for the one skilled (see for example Timothy M. Clay, Mary C. Custer et al. "Efficient Transfer of a Tumor Antigen-Reactive TCR to Human Peripheral Blood Lymphocytes Confers Anti-Tumor Reactivity", The Journal of Immunology, 1999, 163: 507-513).

This has the advantage (this is also valid for the further possibilities described below) that the rate for killing the diseased tissue (e.g. the "tumor-killing rate") can be increased when compared to using immune cells which are not modified by introducing/arranging at least one encapsulated substance.

A second possibility in order to enhance the cytotoxic effector function and/or of the targeting capabilities in said second step is to provide the isolated immune cells with a genetically modified or a chimeric immune cell receptor, wherein preferably the isolated immune cells are cytotoxic T-lymphocytes (CTL) or natural killer cells (NK cells). Especially said second step can be performed with a genetic transfer of chimeric immune globulin (T-cell receptors). This results in "designer immune cells", such as cytotoxic T-lymphocytes or natural killer cells with a genetically modified or chimeric immune cell receptor adapted to increase the recognition of cancer diseases, to activate the immune cells and/or to kill the cancer cells with help of a binding to predetermined markers on the surface. In this case the two-step approach can be performed by firstly activating such immune cells with already enhanced cytotoxic effector function, by then introducing the corresponding substance or medicament into the cells and by finally introducing the correspondingly activated and loaded cells into the mammal body. How a corresponding gene transfer can be realized in detail is well known by the one skilled, see for example Claudia Rössig and Malcolm K. Brenner "Chimeric T-Cell Receptors for the Targeting of Cancer Cells", 2003, Acta Haematologica ; 110: 154-159.

Another possibility in order to enhance the cytotoxic effector function and/or of the targeting capabilities or to perform said second step, respectively, is the following: The isolated immune cells are combined with antibodies and/or fragments of antibodies, wherein preferably bispecific antibodies, trispecific antibodies, diabodies, triabodies and/or tetrabodies or fragments thereof are used in order to improve the capability of the immune cells to recognize the diseased tissue. Therein preferably polyclonal T-cells, which had been isolated from the mammal body, are in a first step loaded with the substance/medicament and thereafter with an antibody, for example with a bispecific or a trispecific antibody, with a diabody, with a triabody and/or a tetrabody or with fragments thereof. Prior to the first step, in an advantageous embodiment, the isolated T-cells are firstly activated ex vivo. Before the described steps, an extension of the T-cells can be performed. Alternatively, also immune cells which are not activated can be loaded with the at least one encapsulated substance/with the medicament. In this case, antibodies (specially bi-specific antibodies or diabodies or other antibodies) are separately injected (preferably before the injection of the cells), so that the activation of the cells is performed in vivo. The activation of these cells in vivo is preferably done at the tumor: E.g. the antibodies can then bind firstly to the tumor and the loaded cells can bind afterwards to the tumor with help of the bi-specific function of the antibodies. Finally, the modified immune cells can be re-introduced into the mammal body with the aim of increasing the cancer recognition rate and/or with the aim to hold the immune cells in the activated state.

How a corresponding combination can be performed is well known for the one skilled, see for example Sergey M. Kipriyanov et al. "Bispecific CD3 x CD19 diabody for T cell-mediated lysis of malignant human B cells", Int. J. Cancer (77), 1998, pages 763-772.

Finally, a fourth method in order to realize the second step of enhancing the cytotoxic effector function and/or the targeting capabilities of the isolated immune cells is to specifically select predetermined immune cell populations out of the extracted immune cells of a patient from which it is known that these immune cell populations migrate to the diseased tissue and/or tumors with high efficiency: Especially T-cells such as tumor-antigen-specific T-lymphocytes or T-cells in combination with re-targeting strategies or monocytes (or the thereof derived macrophages), etc. can be selected.

In all of the cases described above, the selected/isolated immune cells can be, prior to the loading of the medicament/substance and/or to the enhancement of the cytotoxic effector function and prior to the following re-introduction into the human or animal body after modification, expanded if necessary. In the case of the combination with antibodies, especially bispecific antibodies or diabodies, it is possible to use polyclonal T-cells. An expansion is only necessary in specific cases. After the introduction and/or arrangement of the encapsulated substance, the antibodies are combined with the T-cells; thereafter, the so modified immune cells are reinfused. Alternatively, firstly, the antibodies are loaded and then the loading of the substance/medicament is performed.

Alternatively, a two-step approach can be realized in which firstly the bispecific antibodies etc. are introduced into the body, the bispecific antibodies then decorating the tumor etc. and thereafter the T-cells are introduced into the body and the T-cells are held at the tumor with the bispecific antibodies.

Within all of the prescribed methods according to the present invention, the loading of the cells with the encapsulated substance/medicament (i.e. the introduction of the substance/medicament into the mammal immune cell or the arrangement of the encapsulated substance on or at the mammal immune cell) can be performed by incubation of the cells and particles preferably between five minutes and two hours (dependent upon the encapsulation selected) or also by transfection or electroporation.

After reinfusion of the modified immune cells, the immune cells will at first move unaffected by their load according to their natural function within the mammal body and will accumulate in the diseased tissue. Here the cells will exert their intrinsic cytolytic/cytotoxic action i.e. killing of cancer cells. The discharging of the substance/medicament from the encapsulation (after preferably 3 to 14 days) then leads to the initiation of the apoptosis in the carrier cell whereby the releasing of the substance/medicament from the carrier cell into the diseased tissue is facilitated. Besides cytostatics, also other substances, such as angiogenesis inhibitors can be loaded into the immune cells as the encapsulated substances.

When used for diagnostic purposes, e.g. nanoparticles (i.e. particles with a mean diameter of approximately some nanometers up to approximately some micrometers), e.g. ferrites, can be loaded into the immune cells as contrast agent or the like for MRI or the like. By introducing such nanoparticles into immune cells, tumors or metastases can be localized.

The modified immune cells according to the present invention, the cell modification method according to the present invention and the cell modification device according to the present invention can be preferably used for the treatment of cancer diseases, e.g. for the treatment of lymphoma, of colon cancer, of lung cancer and/or of pancreas cancer, and/or ovarian cancer, and or breast cancer and / or brain cancer or the like, in the treatment of virus-infected cells, for the treatment of chronic inflammation, i.e. in chronical inflammation therapy, and/or for the treatment of Alzheimer's disease.

The modified cells according to the present invention (as well as the corresponding cell modification method and cell modification device) have the advantage of an increased cancer treatment capability, especially an increased tumor-killing effect when compared to modified cells according to the state of the art.

Especially by loading the T-cells with the medicament/substance and by releasing the latter within the T-cells, apoptosis of the T-cells is initiated, which leads to an enhanced membrane integrity and an increased release of the medicament.

Hereinafter, an example is given for a cell modification device according to the present invention in which modified immune cells according to the present invention can be generated or in which a cell modification method according to the present invention can be performed.

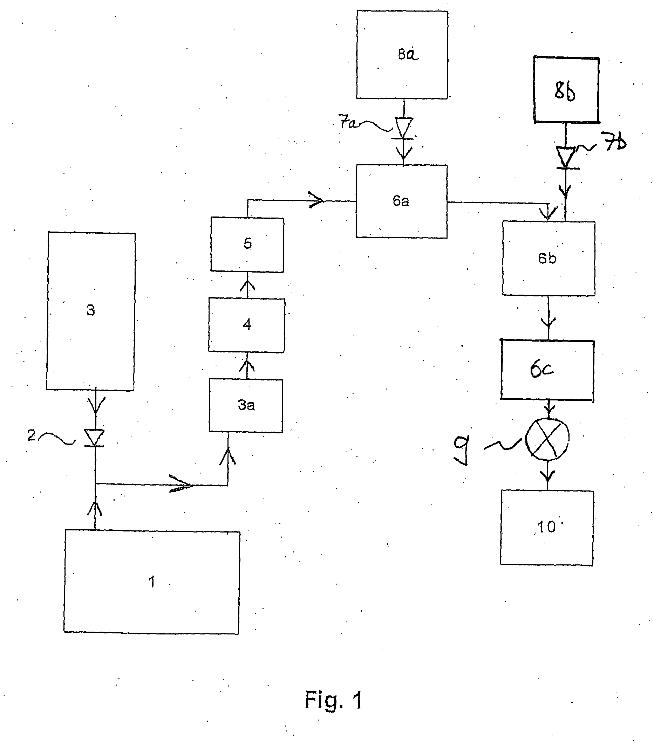

In figure 1 a blood reservoir 1 (e.g. the human blood stream) is schematically shown, from which blood is fed via a filtering device 3a to a cell isolation device 4. In the cell isolation device 4, the desired cells, i.e. the cells to be modified, are selected in a well-known manner. Reference sign 3 shows a filter fluid reservoir 3 from which a filter fluid can be added to the filtering device 3a via a valve 2. The quantity of isolated cells isolated in the isolation device 4 is determined with help of a counting device 5 in a well known manner, e.g. in a device for impedance measurement. In the first loading device 6a, the isolated cells are incubated and loaded with the desired substance/the desired active ingredient: Here, the active ingredient/substance is encapsulated in liposomes, which are added via a lock 7a from an active ingredient/liposome reservoir 8a.

Thus, the kind of carrier used here are liposomes, the used liposome formulation can be as follows:
1) DPPC 30%, cholesterol 40%, DPPG 30%,
2) DPPC 30%, cholesterol 40%, DODAC 30%,
3) DOPE 70%, N-succinylDOPE 30%,
4) DOPE 69.5%, N-succinylDOPE 30% and PEG 0.5% or
5) Cholesterol , 10 to 60 mol%, lecithin 10 to 60 mol%, 5 to 25% of a negative charge carrier out of the group phosphatidylmono, -di, -tri or - tetraglycerol, cholesterol phosphomonoglycerol or cholesterol phosphooligoglycerol.

The carriers can also be core shell nano particles as e. g. poly(DL-lactide-co-glycolide), alternatively grafted with dextran copolymer. Or core shell nano particles with lipid core of lecithin and a medicament, e. g. idarubicin or doxorubicin and a polymer surface of triblock copolymers. The carriers can also be polymer-medicament-conjugates such as for example idarubicin or doxorubicin-PLGA oligomer conjugates.

The substances to be encapsulated can be anthracyclines such as for example doxorubicin, idarubicin, daunorubicin or related compounds such as for example idarubicinol, or the substances to be encapsulated can also be campthotecin and analogues, bleomycin, cisplatin and/or angiogenesis inhibitors and/or angiostatin, endostatin, vitaxin, bevacizuma, recentin.

After the loading of the substance/active ingredient, in a second loading step the cytotoxic effector function of the substance-loaded isolated immune cells is enhanced within the second loading device 6b. This is done by binding a bispecific antibody to the surface of the cell (based on corresponding epitopes and/or eptiopes) the isolated, already substance-loaded immune cells with help of a second reservoir 8b comprising the corresponding bispecific antibodies via a second lock 7b.

In a measurement device 6b the concentration of the active ingredient in the twice loaded cells can be determined. Through the outlet 10 finally the modified cells are fed back to the human blood stream with help of a micropump 9 arranged on the upstream side of the outlet 10.

The described method according to the present invention can be combined with all adoptive and/or cellular immunotherapies.

## Claims

1. Cell modification method for modifying human or mammal immune cells, especially effector cells, outside the human or mammal body,
wherein in a first step at least one encapsulated substance, preferably an active pharmaceutical ingredient, is introduced in and/or arranged on isolated human or mammal immune cells and
wherein in a second step prior to or after the introduction of said at least one substance the cytotoxic effector function of the isolated immune cells is enhanced, and/or the targeting capabilities, preferably the target finding capabilities of the isolated immune cells are enhanced.

2. Cell modification method according to the preceding claim
***characterized in that***
in order to enhance the cytotoxic effector function and/or the targeting capabilities, in said second step at least one immune cell receptor gene, especially a T-cell receptor gene (TCR gene), from a tumour associated antigen (TAA) specific immune cell, especially a TAA specific T-cell, is introduced in the genome of the isolated immune cells, wherein preferably the at least one immune cell receptor gene is introduced by genetic transfer.

3. Cell modification method according to one of the preceding claims
***characterized in that***
in order to enhance the cytotoxic effector function and/or the targeting capabilities, in said second step the isolated immune cells are provided with a genetically modified or a chimeric immune cell receptor, wherein preferably the isolated immune cells are cytotoxic T-lymphocytes (CTL) or natural killer cells (NK cells),
wherein preferably the chimeric immune cell receptor is a chimeric immune globulin T-cell receptor and/or wherein the isolated immune cells are preferably provided with the genetically modified or the chimeric immune cell receptor by genetic transfer.

4. Cell modification method according to one of the preceding claims
***characterized in that***
in order to enhance the cytotoxic effector function and/or the targeting capabilities, in said second step the isolated immune cells are combined with at least one antibody and/or at least one fragment thereof, preferably with at least one bispecific antibody, trispecific antibody, diabody, triabody and/or tetrabody and/or fragments thereof, wherein preferably the at least one antibody and/or fragment thereof is introduced in and/or arranged on the isolated immune cells
and/or wherein preferably the isolated immune cells are monoclonal T-cells or polyclonal T-cells, preferably activated polyclonal T-cells.

5. Cell modification method according to one of the preceding claims
***characterized in that***
in order to enhance the cytotoxic effector function and/or the targeting capabilities, in said second step the following specific types of isolated immune cells is selected as the isolated immune cells: T-cells, especially tumour-antigen specific T-lymphocytes
and/or
**in that** the isolated immune cells are autologous cells, allogenic cells or precursor cells, terminally differentiated effector cells, T-cells, cytotoxic T-cells, especially activated cytotoxic T-cells, TALL-104 cells, C CURE 709 cells and/or cytotoxic T-lymphocytes, tumour-antigen specific T-lymphocytes, NK cells, especially NK-92 cells, monocytes and/or macrophages, especially monocyte-derived macrophages.

6. Cell modification method according to one of the preceding claims
***characterized in that***
prior to the introduction and/or arrangement of the least one encapsulated substance the isolated immune cells are expanded.

7. Cell modification method according to one of the preceding claims
***characterized in that***
at least one of the encapsulated substances is for treatment and comprises a cytostatic drug and/or an angiogenesis inhibitor and/or antibody based therapeutic and/ or therapeutic DNA and/or RNAi-based therapeutic
and/or
at least one of the encapsulated substances is for diagnostics and comprises metallic nanoparticles, especially ferrite nano-particles, i. e. particles with a mean diameter of approximately some ten nm to some µm.

8. Cell modification method according to one of the preceding claims
***characterized in that***
the isolated immune cells are incubated, preferably incubated over a period between approximately five minutes and approximately two hours, in order to introduce and/or arrange the at least one encapsulated substance and/or in order to perform an introduction and/or arrangement used for enhancing the effector function of the isolated immune cells in said second step
and/or
**in that** the at least one encapsulated substance is introduced in and/or arranged on the isolated immune cells by electroporation and/or transfection and/or **in that** an introduction and/or arrangement used for enhancing the effector function of the isolated immune cells in said second step is performed by electroporation and/or transfection
and/or
**in that** the at least one substance is encapsulated thus that the substance is released after a period of between approximately one day and 25 days.

9. Cell modification method according to one of the preceding claims
***characterized in that***
the at least one substance is encapsulated by liposomes, preferably encapsulated liposomes and/or multilaminar liposomes, preferably by using encapsulated liposomes with alginates, preferably with Ba-Alginate, by vesosomes, by virosomes and/or by microspheres comprising porous materials, preferably polylactite or Polymer Polyglycolic-Lactic Acid (PGLA), amylum, apatite and/or pressed polymers Dextran, Agraose, Albumin, Chitosan, Silica or Hollow silica particles, Polyethylenimin, Polyalkylcyanoacrylat, Polymethylmethacrylateparticles, core shell nanoprticles, dendrimers and dendronised polymers, preferably dendrimers and dendronised polymers of which the core molecule is an amine or a sugar with several identical bonding sites for shell molecules available on the surface,
wherein preferably the shells usually alternate between an acid and an amine.

10. Modified human or mammal immune cell, especially effector cell,
comprising at least one encapsulated substance, preferably an active pharmaceutical ingredient, being introduced in and/or arranged on the human or mammal immune cell and
showing an enhanced cytotoxic effector function and/or enhanced targeting capabilities, preferably enhanced target finding capabilities.

11. Modified immune cell according to the preceding claim
***characterized in that***
the enhanced cytotoxic effector function and/or the enhanced targeting capabilities of the modified immune cell is/are effected by the introduction of at least one immune cell receptor gene, especially a T-cell receptor gene (TCR gene), from a tumour associated antigen (TAA) specific immune cell, especially a TAA specific T-cell, in the genome of the immune cell to be modified
and/or
**in that** the enhanced cytotoxic effector function and/or the enhanced targeting capabilities of the modified immune cell is/are effected by the provision of the immune cell to be modified with a genetically modified immune cell receptor or a chimeric immune cell receptor
and/or
**in that** the enhanced cytotoxic effector function and/or the enhanced targeting capabilities of the modified immune cell is/are effected by the combination of the immune cell to be modified with at least one antibody and/or at least one fragment thereof, preferably with at least one bispecific antibody, trispecific antibody, diabody, triabody and/or tetrabody and/or fragments thereof.

12. Modified immune cell according to one of the two preceding claims
***characterized in that***
the immune cell is an autologous cell, an allogenic cell, a precursor cell, a terminally differentiated effector cell, a T-cell, a cytotoxic T-cell, especially an activated cytotoxic T-cell, preferably a TALL-104 cell or a C CURE 709 cell, and/or a cytotoxic T-lymphocyte, a tumour-antigen specific T-lymphocyte, a NK cell, especially a NK-92 cell, a monocyte and/or a macrophage, especially a monocyte-derived macrophage.

13. Cell modification device for modifying human or mammal immune cells, especially effector cells, outside the human or mammal body, the cell modification device comprising:
At least one means (4) for isolating the immune cells and
at least one means (6, 7, 8) adapted for the introduction of and/or the arrangement of at least one encapsulated substance in and/or on the isolated immune cells and in addition adapted for the enhancement of the cytotoxic effector function of the isolated immune cells and/or for the enhancement of the targeting capabilities, preferably the target finding capabilities of the isolated immune cells,
wherein the device preferably also comprises a means (6) for fixing the isolated immune cells prior to the introduction of and/or the arrangement of the at least one encapsulated substance and/or prior to the enhancement of the cytotoxic effector function,
and/or
wherein preferably the cell modification device is adapted to perform the cell modification method according to one of claims 1 to 9.

14. Use of a cell modification device and/or a cell modification method according to one of the preceding claims to enhance the effector function of human or mammal immune cells and/or in cancer therapy, preferably in colon cancer therapy, in lymphoma therapy, in lung cancer therapy, in ovarien cancer therapy, in breast cancer therapy, in brain tumor and/or in pancreas cancer therapy, in chronical inflammation therapy, in Alzheimer disease's therapy and/or in the area of applied immunology or oncology and/or in the area of active and/or autonomous targeting of substances and/or in diagnostic like tumour diagnostics, Alzheimer diagnostic.

15. Use of a modified immune cell according to one of claims 10 to 12 for the manufacture of a medicament for cancer therapy, colon cancer therapy, lymphoma therapy, lung cancer therapy, pancreas cancer therapy, chronical inflammation therapy and/or Alzheimer disease's therapy.
